# EUROPÄISCHE PATENTSCHRIFT

(11) **EP 1 185 670 B1**
(45) Veröffentlichungstag und Bekanntmachung des Hinweises auf die Patenterteilung: **01.09.2004**
(21) Anmeldenummer: 00935197.4
(22) Anmeldetag: 09.06.2000
(51) Int. Cl.: C12N 15/54, C12N 15/82, C12N 15/11, C12N 9/10, C07K 14/415, A01H 5/00, A01H 5/10

(54) **VERFAHREN ZUR ERHÖHUNG DES FETTSÄUREGEHALTS IN PFLANZENSAMEN**
METHOD OF INCREASING THE CONTENT OF FATTY ACIDS IN PLANT SEEDS
PROCEDE POUR AUGMENTER LA TENEUR EN ACIDES GRAS DANS DES GRAINES DE PLANTES

(30) Priorität: 10.06.1999 DE 19926456
(43) Veröffentlichungstag der Anmeldung: 13.03.2002
(73) Patentinhaber: Gesellschaft für Erwerb und Verwertung von Schutzrechten - GVS mbH, 53115 Bonn (DE)
(72) Erfinder: SPENER, Friedrich, D-48147 Münster (DE); SCHÜTT, Burkhardt, Siegfried, D-72108 Rottenburg/Neckar (DE)
(74) Vertreter: Maiwald Patentanwalts GmbH
(86) Internationale Anmeldenummer: PCT/EP2000/005338
(87) Internationale Veröffentlichungsnummer: WO 2000/077224

(56) Entgegenhaltungen:
- WO-A-94/10189
- WO-A-98/46776
- SLABAUGH MARY B ET AL: "Condensing enzymes from Cuphea wrightii associated with medium chain fatty acid biosynthesis." PLANT JOURNAL, Bd. 13, Nr. 5, März 1998 (1998-03), Seiten 611-620, XP002149886 ISSN: 0960-7412 in der Anmeldung erwähnt

### SEQUENZPROTOKOLL

<110> Norddeutsche Pflanzenzucht Hans Georg Lembke KG
<120> Verfahren zur Erhöhung des Fettsäuregehalts in Pflanzensamen
<130> N7095
<140> PCT/EP00/05338
   <141> 2000-06-09
<150> DE19926456.2
   <151> 1999-06-10
<160> 8
<170> Patent In Ver. 2.1
<210> 1
   <211> 2031
   <212> DNA
   <213> Cuphea lanceolata
<400> 1
   ctacttgggt cgcctcagtt ttcaggtgtt ccaatggcgg cggcctcttc catggctgcg 60
   tcaccgttct gtacgtggct cgtagctgct tgcatgtcca cttccttcga aaacaaccca 120
   cgttcgccct ccatcaagcg tctcccccgc cggaggaggg ttctctccca ttgctccctc 180
   cgtggatcca ccttccaatg cctcgtcacc tcacacatcg acccttgcaa tcagaactgc 240
   tcctccgact cccttagctt catcggggtt aacggattcg gatccaagcc attccggtcc 300
   aatcgcggcc accggaggct cggccgtgct tcccattccg gggaggccat ggctgtggct 360
   ctgcaacctg cacaggaagt cgccacgaag aagaaacctg ctatcaagca aaggcgagta 420
   gttgttacag gaatgggtgt ggtgactcct ctaggccatg aacctgatgt tttctacaac 480
   aatctcctag atggagtaag cggcataagt gagatagaga acttcgacag cactcagttt 540
   cccacgagaa ttgccggaga gatcaagtct ttttccacag atggctgggt ggccccaaag 600
   ctctccaaga ggatggacaa gctcatgctt tacttgttga ctgctggcaa gaaagcatta 660
   gcagatgctg gaatcaccga tgatgtgatg aaagagcttg ataaaagaaa gtgtggagtt 720
   ctcattggct ccggaatggg cggcatgaag ttgttctacg atgcgcttga agccctgaaa 780
   atctcttaca ggaagatgaa ccctttttgt gtaccttttg ccaccacaaa tatgggatca 840
   gctatgcttg caatggatct gggatggatg ggtccaaact actctatttc aactgcctgt 900
   gcaacaagta atttctgtat actgaatgct gcaaaccaca taatcagagg cgaagctgac 960
   atgatgcttt gtggtggctc ggatgcggtc attataccta tcggtttggg aggttttgtg 1020
   gcgtgccgag ctttgtcaca gaggaataat gaccctacca aagcttcgag accatgggat 1080
   agtaatcgtg atggatttgt aatgggcgaa ggagctggag tgttacttct cgaggagtta 1140
   gagcatgcaa agaaaagagg tgcaaccatt tatgcagaat tttagggggg cagtttcact 1200
   tgcgatgcct accacatgac cgagcctcac cctgaaggag ctggagtgat cctctgcata 1260
   gagaaggcca tggctcaggc cggagtctct agagaagatg taaattacat aaatgcccat 1320
   gcaacttcca ctcctgctgg agatatcaaa gaataccaag ctctcgccca ctgtttcggc 1380
   caaaacagcg agctgagagt gaattccact aaatcgatga tcggtcatct tcttggagca 1440
   gctggtggcg tagaagcagt tactgtaatt caggcgataa ggactgggtg gatccatcca 1500
   aatcttaatt tggaagaccc ggacaaagcc gtggatgcaa aatttctcgt gggacctgag 1560
   aaggagagac tgaatgtcaa ggtcggtttg tccaattcat ttgggttcgg tgggcataac 1620
   tcgtctatac tcttcgcccc ttacaattag gtatgtttcg tgtggaattc ttcgctcaat 1680
   ggatgccaaa gttttttaga actcctgcac gttagtagct tatgtctctg gacatggaaa 1740
   tggaatttgg gttggaagct gtagccagaa gactcagaac catgatagac cgagcactca 1800
   cgacgatgcc aaagatactc cttgccggta ttgttgttaa gagtccnctg tttgtccctt 1860
   ttttcttttc ctctcttcct catcgatatt agtcgcactt ttgagctttt gatcaagcta 1920
   gtgaagatac aaagatacct cgggcacgta gttgcttggt ttgccacaat ctgtaaaact 1980
   cgggactggt ttagtttcag tgtgtttatc ctaaaaaaaa aaaaaaaaaa a          2031
<210> 2
   <211> 538
   <212> PRT
   <213> Cuphea lanceolata
<400> 2
   Met Ala Ala Ala Ser Ser Met Ala Ala Ser Pro Phe Cys Thr Trp Leu
   1 5 10 15
   Val Ala Ala Cys Met Ser Thr Ser Phe Glu Asn Asn Pro Arg Ser Pro
   20 25 30
   Ser Ile Lys Arg Leu Pro Arg Arg Arg Arg Val Leu Ser His Cys Ser
   35 40 45
   Leu Arg Gly Ser Thr Phe Gln Cys Leu Val Thr Ser His Ile Asp Pro
   50 55 60
   Cys Asn Gln Asn Cys Ser Ser Asp Ser Leu Ser Phe Ile Gly Val Asn
   65 70 75 80
   Gly Phe Gly Ser Lys Pro Phe Arg Ser Asn Arg Gly His Arg Arg Leu
   85 90 95
   Gly Arg Ala Ser His Ser Gly Glu Ala Met Ala Val Ala Leu Gln Pro
   100 105 110
   Ala Gln Glu Val Ala Thr Lys Lys Lys Pro Ala Ile Lys Gln Arg Arg
   115 120 125
   Val Val Val Thr Gly Met Gly Val Val Thr Pro Leu Gly His Glu Pro
   130 135 140
   Asp Val Phe Tyr Asn Asn Leu Leu Asp Gly Val Ser Gly Ile Ser Glu
   145 150 155 160
   Ile Glu Asn Phe Asp Ser Thr Gln Phe Pro Thr Arg Ile Ala Gly Glu
   165 170 175
   Ile Lys Ser Phe Ser Thr Asp Gly Trp Val Ala Pro Lys Leu Ser Lys
   180 185 190
   Arg Met Asp Lys Leu Met Leu Tyr Leu Leu Thr Ala Gly Lys Lys Ala
   195 200 205
   Leu Ala Asp Ala Gly Ile Thr Asp Asp Val Met Lys Glu Leu Asp Lys
   210 215 220
   Arg Lys Cys Gly Val Leu Ile Gly Ser Gly Met Gly Gly Met Lys Leu
   225 230 235 240
   Phe Tyr Asp Ala Leu Glu Ala Leu Lys Ile Ser Tyr Arg Lys Met Asn
   245 250 255
   Pro Phe Cys Val Pro Phe Ala Thr Thr Asn Met Gly Ser Ala Met Leu
   260 265 270
   Ala Met Asp Leu Gly Trp Met Gly Pro Asn Tyr Ser Ile Ser Thr Ala
   275 280 285
   Cys Ala Thr Ser Asn Phe Cys Ile Leu Asn Ala Ala Asn His Ile Ile
   290 295 300
   Arg Gly Glu Ala Asp Met Met Leu Cys Gly Gly Ser Asp Ala Val Ile
   305 310 315 320
   Ile Pro Ile Gly Leu Gly Gly Phe Val Ala Cys Arg Ala Leu Ser Gln
   325 330 335
   Arg Asn Asn Asp Pro Thr Lys Ala Ser Arg Pro Trp Asp Ser Asn Arg
   340 345 350
   Asp Gly Phe Val Met Gly Glu Gly Ala Gly Val Leu Leu Leu Glu Glu
   355 360 365
   Leu Glu His Ala Lys Lys Arg Gly Ala Thr Ile Tyr Ala Glu Phe Leu
   370 375 380
   Gly Gly Ser Phe Thr Cys Asp Ala Tyr His Met Thr Glu Pro His Pro
   385 390 395 400
   Glu Gly Ala Gly Val Ile Leu Cys Ile Glu Lys Ala Met Ala Gln Ala
   405 410 415
   Gly Val Ser Arg Glu Asp Val Asn Tyr Ile Asn Ala His Ala Thr Ser
   420 425 430
   Thr Pro Ala Gly Asp Ile Lys Glu Tyr Gln Ala Leu Ala His Cys Phe
   435 440 445
   Gly Gln Asn Ser Glu Leu Arg Val Asn Ser Thr Lys Ser Met Ile Gly
   450 455 460
   His Leu Leu Gly Ala Ala Gly Gly Val Glu Ala Val Thr Val Ile Gln
   465 470 475 480
   Ala Ile Arg Thr Gly Trp Ile His Pro Asn Leu Asn Leu Glu Asp Pro
   485 490 495
   Asp Lys Ala Val Asp Ala Lys Phe Leu Val Gly Pro Glu Lys Glu Arg
   500 505 510
   Leu Asn Val Lys Val Gly Leu Ser Asn Ser Phe Gly Phe Gly Gly His
   515 520 525
   Asn Ser Ser Ile Leu Phe Ala Pro Tyr Asn
   530 535
<210> 3
   <211> 1284
   <212> DNA
   <213> Brassica napus
<400> 3
   atggagaagg atgctatggt tagcaagaaa cctcctttcg agccacgccg agttgttgtc 60
   actggcatgg gagttgaaac gccactaggt cacgaccctc atacttttta tgacaacctg 120
   cttctaggca acagtggtat aagccatata gagagtttcg actgttctgc atttcccact 180
   agaatcgctg gagagattaa atctttttcg acccaaggat tggttgctcc taaactttcc 240
   aaaaggatgg acaagttcat gctttacctt ctcaccgccg gcaagaaggc gttggaggat 300
   ggtgtggtga ctgaggatgt gatggcagag ttcgacaaat caagatgtgg tgtcttgatt 360
   ggctcagcaa tgggaggcat gaaggtcttc tacgatgcgc ttgaagcttt gaaaatctct 420
   tacaggaaga tgagcccttt ttgtgtacct tttgccacca caaacatggg ttccgctatg 480
   cttgccttgg atctgggatg gatgggtcca aactactcta tttcaaccgc atgtgccacg 540
   ggaaacttct gtattctcaa tgcagcaaac cacatcacaa gaggtgaagc tgatgtaatg 600
   ctctgcggtg gctctgactc agttattatt ccaatagggt tgggaggttt tgttgcctgc 660
   cgggctcttt cagaaaataa tgatgatccc accaaagctt ctcgtccttg ggatagtaac 720
   cgagatggtt ttgttatggg agagggagcc ggagttctac ttttagaaga acttgagcat 780
   gccaagaaaa gaggagcaac tatatacgca gagttccttg ggggtagttt cacatgtgat 840
   gcataccata taaccgaacc acgtcctgat ggtgctggtg tcattctcgc tatcgagaaa 900
   gcgttagctc atgccgggat ttctaaggaa gacataaatt acgtgaatgc tcatgctacc 960
   tctacaccag ctggagacct taaggagtac cacgcccttt ctcactgttt tggccaaaat 1020
   cctgagctaa gggtaaactc aacaaaatct atgattggac acttgctggg agcttctggg 1080
   gccgtggagg ctgttgcaac cgttcaggca ataaagacag gatgggttca tccaaatatc 1140
   aacctcgaga atccagacaa agcagtggat acaaagcttc tggtgggtct taagaaggag 1200
   aggctggata tcaaagcagc tttgtcaaac tctttcggct ttggtggcca gaactctagc 1260
   atcattttcg cgccctacaa ctga                                       1284
<210> 4
   <211> 427
   <212> PRT
   <213> Brassica napus
<400> 4
   Met Glu Lys Asp Ala Met Val Ser Lys Lys Pro Pro Phe Glu Pro Arg
    1               5                  10                  15
   Arg Val Val Val Thr Gly Met Gly Val Glu Thr Pro Leu Gly His Asp
               20                  25                  30
   Pro His Thr Phe Tyr Asp Asn Leu Leu Leu Gly Asn Ser Gly Ile Ser
           35                  40                  45
   His Ile Glu Ser Phe Asp Cys Ser Ala Phe Pro Thr Arg Ile Ala Gly
       50                  55                  60
   Glu Ile Lys Ser Phe Ser Thr Gln Gly Leu Val Ala Pro Lys Leu Ser
   65                  70                  75                  80
   Lys Arg Met Asp Lys Phe Met Leu Tyr Leu Leu Thr Ala Gly Lys Lys
                   85                  90                  95
   Ala Leu Glu Asp Gly Val Val Thr Glu Asp Val Met Ala Glu Phe Asp
               100                 105                 110
   Lys Ser Arg Cys Gly Val Leu Ile Gly Ser Ala Met Gly Gly Met Lys
           115                 120                 125
   Val Phe Tyr Asp Ala Leu Glu Ala Leu Lys Ile Ser Tyr Arg Lys Met
       130                 135                 140
   Ser Pro Phe Cys Val Pro Phe Ala Thr Thr Asn Met Gly Ser Ala Met
   145                 150                 155                 160
   Leu Ala Leu Asp Leu Gly Trp Met Gly Pro Asn Tyr Ser Ile Ser Thr
                   165                 170                 175
   Ala Cys Ala Thr Gly Asn Phe Cys Ile Leu Asn Ala Ala Asn His Ile
   180 185 190
   Thr Arg Gly Glu Ala Asp Val Met Leu Cys Gly Gly Ser Asp Ser Val
   195 200 205
   Ile Ile Pro Ile Gly Leu Gly Gly Phe Val Ala Cys Arg Ala Leu Ser
   210 215 220
   Glu Asn Asn Asp Asp Pro Thr Lys Ala Ser Arg Pro Trp Asp Ser Asn
   225 230 235 240
   Arg Asp Gly Phe Val Met Gly Glu Gly Ala Gly Val Leu Leu Leu Glu
   245 250 255
   Glu Leu Glu His Ala Lys Lys Arg Gly Ala Thr Ile Tyr Ala Glu Phe
   260 265 270
   Leu Gly Gly Ser Phe Thr Cys Asp Ala Tyr His Ile Thr Glu Pro Arg
   275 280 285
   Pro Asp Gly Ala Gly Val Ile Leu Ala Ile Glu Lys Ala Leu Ala His
   290 295 300
   Ala Gly Ile Ser Lys Glu Asp Ile Asn Tyr Val Asn Ala His Ala Thr
   305 310 315 320
   Ser Thr Pro Ala Gly Asp Leu Lys Glu Tyr His Ala Leu Ser His Cys
   325 330 335
   Phe Gly Gln Asn Pro Glu Leu Arg Val Asn Ser Thr Lys Ser Met Ile
   340 345 350
   Gly His Leu Leu Gly Ala Ser Gly Ala Val Glu Ala Val Ala Thr Val
   355 360 365
   Gln Ala Ile Lys Thr Gly Trp Val His Pro Asn Ile Asn Leu Glu Asn
   370 375 380
   Pro Asp Lys Ala Val Asp Thr Lys Leu Leu Val Gly Leu Lys Lys Glu
   385 390 395 400
   Arg Leu Asp Ile Lys Ala Ala Leu Ser Asn Ser Phe Gly Phe Gly Gly
   405 410 415
   Gln Asn Ser Ser Ile Ile Phe Ala Pro Tyr Asn
   420 425
<210> 5
   <211> 1254
   <212> DNA
   <213> Brassica napus
<400> 5
   atggagaaag acgccatggt aaacaagcca cgccgagttg ttgtcactgg catgggagtt 60
   gaaacaccac taggtcacga ccctcatact ttttatgaca acttgctaca aggcaaaagt 120
   ggtataagcc atatagagag tttcgactgt tctgcatttc ccactagaat cgctggggag 180
   attaaatctt tttcgaccga cggattggtt gctcctaaac tttccaaaag gatggacaag 240
   ttcatgctct accttctaac agctggcaag aaggcgttgg aggatggtgg ggtgactggg 300
   gatgtgatgg cagagttcga caaagcaaga tgtggtgtct tgattggctc agcaatggga 360
   ggcatgaagg tcttctacga tgcgcttgaa gctttgaaaa tctcttacag gaagatgaat 420
   tttgccacca caaacatggg ttccgctatg cttgccttgg atctgggatg gatgggtcca 480
   aactactcta tttcaaccgc atgtgccacg ggaaacttct gtattcacaa tgcggcaaac 540
   cacattacta gaggtgaagc tgatgtaatg ctctgtggtg gctctgactc agttattatt 600
   ccaatagggt tgggaggttt tgttgcctgc cgggctcttt cagaaaataa tgatgatccc 660
   accaaagctt ctcgtccttg ggatagtaac cgagatggtt ttgttatggg agagggagcc 720
   ggagttctac ttttagaaga acttgagcat gccaagaaaa gaggagcaac tatatacgca 780
   gagttccttg gggtagtttt cacatgggat gcatatcata ttaccgaacc acatcctgat 840
   ggtgctggtg tcattctcgc tatcgagaaa gcattagctc atgccgggat ttctaaggaa 900
   gacataaatt acgtgaatgc tcatgctacc tctacaccag ctggagacct taaggagtac 960
   cacgcccttt ctcactgttt tggccaaaat cctgagctaa gggtaaactc aacaaaatct 1020
   atgattggac acttgctggg agcttctggg gccgtggagg ctgttgcaac cgttcaggca 1080
   ataaagacag gatgggttca tccaaattac aacctcgaga atccagacaa agcagtggat 1140
   acaaagcttc tggtgggtct taagaaggag agactggata tcaaagcagc tttgtcaaac 1200
   tctttcggct ttggtggcca gaactctagc atcattttcg ccccctacaa ttga       1254
<210> 6
   <211> 417
   <212> PRT
   <213> Brassica napus
<400> 6
   Met Glu Lys Asp Ala Met Val Asn Lys Pro Arg Arg Val Val Val Thr
    1               5                  10                  15
   Gly Met Gly Val Glu Thr Pro Leu Gly His Asp Pro His Thr Phe Tyr
               20                  25                  30
   Asp Asn Leu Leu Gln Gly Lys Ser Gly Ile Ser His Ile Glu Ser Phe
           35                  40                  45
   Asp Cys Ser Ala Phe Pro Thr Arg Ile Ala Gly Glu Ile Lys Ser Phe
       50                  55                  60
   Ser Thr Asp Gly Leu Val Ala Pro Lys Leu Ser Lys Arg Met Asp Lys
   65 70 75 80
   Phe Met Leu Tyr Leu Leu Thr Ala Gly Lys Lys Ala Leu Glu Asp Gly
   85 90 95
   Gly Val Thr Gly Asp Val Met Ala Glu Phe Asp Lys Ala Arg Cys Gly
   100 105 110
   Val Leu Ile Gly Ser Ala Met Gly Gly Met Lys Val Phe Tyr Asp Ala
   115 120 125
   Leu Glu Ala Leu Lys Ile Ser Tyr Arg Lys Met Asn Phe Ala Thr Thr
   130 135 140
   Asn Met Gly Ser Ala Met Leu Ala Leu Asp Leu Gly Trp Met Gly Pro
   145 150 155 160
   Asn Tyr Ser Ile Ser Thr Ala Cys Ala Thr Gly Asn Phe Cys Ile His
   165 170 175
   Asn Ala Ala Asn His Ile Thr Arg Gly Glu Ala Asp Val Met Leu Cys
   180 185 190
   Gly Gly Ser Asp Ser Val Ile Ile Pro Ile Gly Leu Gly Gly Phe Val
   195 200 205
   Ala Cys Arg Ala Leu Ser Glu Asn Asn Asp Asp Pro Thr Lys Ala Ser
   210 215 220
   Arg Pro Trp Asp Ser Asn Arg Asp Gly Phe Val Met Gly Glu Gly Ala
   225 230 235 240
   Gly Val Leu Leu Leu Glu Glu Leu Glu His Ala Lys Lys Arg Gly Ala
   245 250 255
   Thr Ile Tyr Ala Glu Phe Leu Gly Gly Ser Phe Thr Trp Asp Ala Tyr
   260 265 270
   His Ile Thr Glu Pro His Pro Asp Gly Ala Gly Val Ile Leu Ala Ile
   275 280 285
   Glu Lys Ala Leu Ala His Ala Gly Ile Ser Lys Glu Asp Ile Asn Tyr
   290 295 300
   Val Asn Ala His Ala Thr Ser Thr Pro Ala Gly Asp Leu Lys Glu Tyr
   305 310 315 320
   His Ala Leu Ser His Cys Phe Gly Gln Asn Pro Glu Leu Arg Val Asn
   325 330 335
   Ser Thr Lys Ser Met Ile Gly His Leu Leu Gly Ala Ser Gly Ala Val
   340 345 350
   Glu Ala Val Ala Thr Val Gln Ala Ile Lys Thr Gly Trp Val His Pro
   355 360 365
   Asn Tyr Asn Leu Glu Asn Pro Asp Lys Ala Val Asp Thr Lys Leu Leu
   370 375 380
   Val Gly Leu Lys Lys Glu Arg Leu Asp Ile Lys Ala Ala Leu Ser Asn
   385 390 395 400
   Ser Phe Gly Phe Gly Gly Gln Asn Ser Ser Ile Ile Phe Ala Pro Tyr
   405 410 415
   Asn
<210> 7
   <211> 1278
   <212> DNA
   <213> Cuphea lanceolata
<400> 7
   acgatctcag ctccaaagcg cgagtccgac cccaagaagc gtgtcgtcat caccggcatg 60
   ggcctcgtct ccatattcgg atccgacgtc gacgcctact acgacaagct gctctccggc 120
   gagagcggca tcagcttaat cgaccgcttc gacgcttcca agttccccac caggttcggc 180
   ggccagatcc gtggcttcaa cgcgacgggc tacatcgacg gcaagaacga ccggcggctc 240
   gacgattgcc tccgttactg cattgtcgcc ggcaagaagg ctctcgaaga cgccgatctc 300
   gccggccaat ccctctccaa gattgataag gagagggccg gagtgctagt tggaaccggt 360
   atgggtggcc taactgtctt ctctgacggg gttcagaatc tcatcgagaa aggtcaccgg 420
   aagatctccc cgtttttcat tccatatgcc attacaaaca tggggtctgc cctgcttgcc 480
   atcgacttgg gtctgatggg cccaaactat tcgatttcaa ctgcatgtgc tacttccaac 540
   tactgctttt atgctgctgc caatcatatc cgccgaggtg aggctgacct gatgattgct 600
   ggaggaactg aggctgcgat cattccaatt ggtttaggag gattcgttgc ctgcagggct 660
   ttatctcaaa ggaatgatga ccctcagact gcctcaaggc cgtgggataa ggaccgtgat 720
   ggttttgtga tgggtgaagg ggctggagta ttggttatgg agagcttgga acatgcaatg 780
   aaacggggag cgccgattat tgcagaatat ttgggaggtg cagtcaactg tgatgcttat 840
   catatgactg atccaagggc tgatgggctt ggtgtctcct catgcattga gagcagtctc 900
   gaagatgctg gggtctcacc tgaagaggtc aattacataa atgctcatgc gacttctact 960
   cttgctgggg atcttgccga gataaatgcc atcaagaagg ttttcaagaa caccaaggaa 1020
   atcaaaatca acgcaactaa gtcaatgatc ggccactgtc ttggagcatc aggaggtctt 1080
   gaagccatcg caaccattaa gggaataact tccggctggc ttcatcccag cattaatcaa 1140
   ttcaatcccg agccatcggt ggacttcgac actgttgcca acaagaagca gcaacatgaa 1200
   gtcaacgtcg ctatctcaaa ttcattcgga tttggaggcc acaactcagt tgtggctttc 1260
   tcagctttca agccatga 1278
<210> 8
   <211> 425
   <212> PRT
   <213> Cuphea lanceolata
<400> 8
   Thr Ile Ser Ala Pro Lys Arg Glu Ser Asp Pro Lys Lys Arg Val Val
   1               5                   10                  15
   Ile Thr Gly Met Gly Leu Val Ser Ile Phe Gly Ser Asp Val Asp Ala
               20                  25                  30
   Tyr Tyr Asp Lys Leu Leu Ser Gly Glu Ser Gly Ile Ser Leu Ile Asp
           35                  40                  45
   Arg Phe Asp Ala Ser Lys Phe Pro Thr Arg Phe Gly Gly Gln Ile Arg
       50                  55                  60
   Gly Phe Asn Ala Thr Gly Tyr Ile Asp Gly Lys Asn Asp Arg Arg Leu
   65                  70                  75                  80
   Asp Asp Cys Leu Arg Tyr Cys Ile Val Ala Gly Lys Lys Ala Leu Glu
                   85                  90                  95
   Asp Ala Asp Leu Ala Gly Gln Ser Leu Ser Lys Ile Asp Lys Glu Arg
               100                 105                 110
   Ala Gly Val Leu Val Gly Thr Gly Met Gly Gly Leu Thr Val Phe Ser
           115                 120                 125
   Asp Gly Val Gln Asn Leu Ile Glu Lys Gly His Arg Lys Ile Ser Pro
       130                 135                 140
   Phe Phe Ile Pro Tyr Ala Ile Thr Asn Met Gly Ser Ala Leu Leu Ala
   145                 150                 155                 160
   Ile Asp Leu Gly Leu Met Gly Pro Asn Tyr Ser Ile Ser Thr Ala Cys
                   165                 170                 175
   Ala Thr Ser Asn Tyr Cys Phe Tyr Ala Ala Ala Asn His Ile Arg Arg
               180                 185                 190
   Gly Glu Ala Asp Leu Met Ile Ala Gly Gly Thr Glu Ala Ala Ile Ile
           195                 200                 205
   Pro Ile Gly Leu Gly Gly Phe Val Ala Cys Arg Ala Leu Ser Gln Arg
   210 215 220
   Asn Asp Asp Pro Gln Thr Ala Ser Arg Pro Trp Asp Lys Asp Arg Asp
   225 230 235 240
   Gly Phe Val Met Gly Glu Gly Ala Gly Val Leu Val Met Glu Ser Leu
   245 250 255
   Glu His Ala Met Lys Arg Gly Ala Pro Ile Ile Ala Glu Tyr Leu Gly
   260 265 270
   Gly Ala Val Asn Cys Asp Ala Tyr His Met Thr Asp Pro Arg Ala Asp
   275 280 285
   Gly Leu Gly Val Ser Ser Cys Ile Glu Ser Ser Leu Glu Asp Ala Gly
   290 295 300
   Val Ser Pro Glu Glu Val Asn Tyr Ile Asn Ala His Ala Thr Ser Thr
   305 310 315 320
   Leu Ala Gly Asp Leu Ala Glu Ile Asn Ala Ile Lys Lys Val Phe Lys
   325 330 335
   Asn Thr Lys Glu Ile Lys Ile Asn Ala Thr Lys Ser Met Ile Gly His
   340 345 350
   Cys Leu Gly Ala Ser Gly Gly Leu Glu Ala Ile Ala Thr Ile Lys Gly
   355 360 365
   Ile Thr Ser Gly Trp Leu His Pro Ser Ile Asn Gln Phe Asn Pro Glu
   370 375 380
   Pro Ser Val Asp Phe Asp Thr Val Ala Asn Lys Lys Gln Gln His Glu
   385 390 395 400
   Val Asn Val Ala Ile Ser Asn Ser Phe Gly Phe Gly Gly His Asn Ser
   405 410 415
   Val Val Ala Phe Ser Ala Phe Lys Pro
   420 425

## Beschreibung

Die vorliegende Erfindung betrifft Verfahren zur Erhöhung des Fettsäuregehalts, insbesondere des Gehalts an kurz kettigen Fettsäuren, in Triglyceriden von Pflanzensamen, umfassend die Expression eines Proteins mit der Aktivität einer β-Ketoacyl-ACP-Synthese II (KASII) in transgenen Pflanzensamen.

Die Fettsäure- und Triacylglyceridbiosynthese lassen sich aufgrund der Kompartimentierung als getrennte Biosynthesewege, jedoch im Hinblick auf das Endprodukt als ein Biosyntheseweg ansehen. Die de növo Biosynthese von Fettsäuren erfolgt in den Plastiden und wird im wesentlichen von drei Enzymen bzw. Enzymsystemen katalysiert, nämlich der Acetyl-CoA-Carboxylase, der Fettsäuresynthase und der Acetyl-ACP-Thioesterase. Die Endprodukte dieser Reaktionsfolge sind in den meisten Organismen Palmitat, Stearat und, nach einer Desaturierung, Oleat.

Die Fettsäuresynthase besteht aus einem Enzymkomplex dissoziierbarer Einzelenzyme mit den einzelnen Enzymen Acetyl-ACP-Transacylase, Malonyl-ACP-Transacylase, β-Ketoacyl-ACP-Synthasen (Acyl-Malonyl-ACP-kondensierende Enzyme), β-Ketoacyl-ACP-Reduktase, 3-Hydroxyacyl-ACP-Dehydratase und Enoyl-ACP-Reduktase.

Die Verlängerungsphase der Fettsäuresynthese beginnt mit der Bildung von Acetyl-ACP und Malonyl-ACP. Die Acetyl-Transacylase und die Malonyl-Transacylase katalysieren diese Reaktionen. Acetyl-ACP und Malonyl-ACP reagieren miteinander unter Bildung von Acetoacetyl-ACP, diese Kondensationsreaktion wird vom Acyl-Malonyl-Acetylkondensierenden Enzym katalysiert. In den nächsten drei Schritten der Fettsäuresynthese wird die Ketogruppe am C-3 zu einer Methylengruppe reduziert, wobei zuerst das Acetoacetyl-ACP zu D-3-Hydroxybutyryl-ACP reduziert wird und anschließend aus dem D-3-Hydroxybutyryl-ACP durch Wasserabspaltung Crotonyl-ACP entsteht. Im letzten Schritt des Zyklus wird Crotonyl-ACP zu Butyryl-ACP reduziert, wodurch der erste Verlängerungszyklus abgeschlossen ist. In der zweiten Runde der Fettsäuresynthese kondensiert Butyryl-ACP mit Malonyl-ACP zu C₆-β-Ketoacyl-ACP. Anschließende Reduktion, Wasserabspaltung und eine zweite Reduktion überführen das C₆-β-Ketoacyl-ACP in C₆-Acyl-ACP, das für eine dritte Verlängerungsrunde bereitgestellt wird. Diese Verlängerungszyklen laufen weiter bis zum C₁₆-Acyl-ACP. Dieses Produkt ist kein Substrat mehr für das kondensierende Enzym. Es wird vielmehr zu Palmitat und ACP hydrolisiert.

Im sogenannten Kennedy-Pathway erfolgt dann im Cytoplasma am endoplasmatischen Retikulum die Triacylglyceridbiosynthese aus Glycerin-3-Phosphat und Fettsäuren, die als Acyl-CoA-Substrat vorliegen.

Unter den Begriff Fettsäuren fallen gesättigte oder ungesättigte, kurz-, mittel- oder langkettige, geradkettige oder verzweigte, geradzahlige oder ungeradzahlige Fettsäuren. Als kurzkettige Fettsäuren werden allgemein die Fettsäuren mit bis zu 6 C-Atomen bezeichnet. Hierzu zählen somit Buttersäure, Valeriansäure und Hexansäure. Unter die Bezeichnung mittelkettige Fettsäuren fallen C₈- bis C₁₄-Fettsäuren, also in erster Linie Oktansäure, Caprinsäure, Laurinsäure und Myristinsäure. Schließlich zählt man zu den langkettigen Fettsäuren solche mit mindestens 16 C-Atomen, also vor allem Palmitinsäure, Stearinsäure, Ölsäure, Linolsäure und Linolensäure.

Fettsäuren, die in sämtlichen pflanzlichen und tierischen Fetten und vor allem in pflanzlichen Ölen und Fischölen vorkommen, sind vielseitig einsetzbar. Zum Beispiel kann ein Mangel an essentiellen Fettsäuren, also Fettsäuren, die im Organismus nicht synthetisiert werden können und daher mit der Nahrung zugeführt werden müssen, zu Hautveränderungen und Wachstumsstörungen führen, weshalb Fettsäuren u.a. bei Ekzemen, Psoriasis, Verbrennungen u.ä., sowie auch in der Kosmetik eingesetzt werden. Weiter finden Fettsäuren und Öle in Wasch- und Reinigungsmitteln, als Detergentien, als Farbzusätze, Schmier- und Gleitstoffe, Verarbeitungshilfen, Emulgationshilfen, Hydrauliköle und als Trägeröle in pharmazeutischen und kosmetischen Erzeugnissen Anwendung. Als nachwachsende Rohstoffe im chemischtechnischen Sektor werden natürliche Fette und Öle tierischen (z.B. Talg) und pflanzlichen (z.B. Kokosnuß-, Palmkem- oder Rapsöl) Ursprungs eingesetzt. Die Einsatzbereiche pflanzlicher Öle wurden in den letzten zwanzig Jahren deutlich erweitert. Mit steigendem Umweltbewußtsein entwickelte man beispielsweise umweltverträgliche Schmierstoffe und Hydrauliköle. Weitere Anwendungen finden Fettsäuren und Fette als Nahrungsmittel bzw. Nahrungsmittelzusatz, z.B. in der parenteralen Ernährung, Backhilfen, in der Baby-, Senoren- und Sportlerkost, in Schokoladenmassen, Kakaopulver und als Backfette, zur Herstellung von Seifen, Salben, Kerzen, Malerfarben und Textilfarben, Firnissen, Heiz- und Beleuchtungsmitteln.

Pflanzenzüchterische Ziele sind insbesondere die Erhöhung des Gehalts von Fettsäuren in Sainenölen. So besteht ein Zuchtziel in Bezug auf Industrieraps und alternative Produktionsbereiche für die Landwirtschaft in der Gewinnung von Rapsöl mit Fettsäuren mittlerer Kettenlänge, hauptsächlich C₁₂, da diese besonders begehrt sind in der Tensidherstellung. Neben dem Gedanken, pflanzliche Öle als Industrierohstoffe zu verwenden, besteht die Möglichkeit, sie als Biotreibstoff einzusetzen.

Es besteht daher ein Bedarf an Bereitstellung von Fettsäuren, die z.B. als Grundstoffe für Weichmacher, Schmierstoffe, Pestizide, Tenside, Kosmetika usw. industriell einseztbar und/oder nahrungsmitteltechnologisch wertvoll sind. Eine Möglichkeit zur Bereitstellung von Fettsäuren besteht in der Extraktion der Fettsäuren aus Pflanzen, die besonders hohe Gehalte dieser Fettsäuren aufweisen. Die Erhöhung von Gehalten an beispielsweise mittelkettigen Fettsäuren auf klassischem Wege, also durch Züchtung von Pflanzen, die in erhöhtem Maße diese Fettsäuren produzieren, konnte bisher nur bedingt erreicht werden.

Es ist daher eine Aufgabe der Erfindung, Verfahren zur Erhöhung des Gehalts an Fettsäuren, insbesondere mittelkettigen und kurzkettigen Fettsäuren in Pflanzensamen bereitzustellen.

Zur Lösung dieser Aufgabe dienen die Merkmale von Anspruch 1.

Vorteilhafte Ausgestaltungen sind in den Unteransprüchen definiert.

Es wurde überraschenderweise gefunden, daß pflanzliche Enzyme mit der Aktivität einer β-Ketoacyl-ACP-Synthase II nicht, wie bisher angenommen, nur längerkettige Fettsäuren synthetisieren, also C₁₄- und C₁₆-Acyl-ACP-Substrate verwenden, sondern darüberhinaus eine Spezifität für C₄- und C₆-Substrate besitzen. Dies bedeutet, daß eine Erhöhung des Gehalts an kurzkettigen Fettsäuren in Pflanzen mittels Übertragung von Sequenzen für KASII möglich ist.

Die Erfindung betrifft daher ein Verfahren zur Erhöhung des Gehalts an kurzkettigen Fettsäuren in Pflanzensamen, umfassend die Schritte:
a)
   Herstellung einer Nukleinsäuresequenz, mindestens umfassend die folgenden Bestandteile, die in der 5'-3'-Orientierung aneinandergereiht sind:
   ein in Pflanzen, insbesondere in embryonalen Geweben, aktiver Promotor,
   mindestens eine Nukleinsäuresequenz, die für ein Protein mit der Aktivitität einer β-Ketoacyl-ACP-Synthase II oder ein aktives Fragment davon kodiert und
   gegebenenfalls ein Terminationssignal für die Termination der Transkription und die Addition eines Poly-A-Schwanzes an das entsprechende Transkript, sowie gegebenenfalls davon abgeleitete DNA-Sequenzen;
b)
   Übertragung der Nukleinsäuresequenz aus a) auf pflanzliche Zellen und
c)
   gegebenenfalls Regeneration vollständig transformierter Pflanzen und, falls erwünscht, Vermehrung der Pflanzen.

In einer bevorzugten Ausführungsform werden zusätzlich zu KAS II-Sequenzen DNA-Konstrukte übertragen, die eine Suppression endogener KAS I-Sequenzen gewährleisten, also z.B. Antisense- oder Co-Suppressions-Konstrukte gegen KAS I. Da die endogene KASI-Aktivität natürlicherweise eine Elongation kurzkettiger Substrate zu mittelkettigen Fettsäuren bewirkt, wird durch Unterdrückung der endogenen KAS I-Aktivität eine effiziente Bereitstellung und Akkumulation von kurzkettigen Fettsäuren erreicht.

In einer bevorzugten Ausführungsform werden die erfindungsgemäßen KAS-Sequenzen unter Kontrolle samenspezifischer Regulationselemente, insbesondere Promotoren, in Pflanzenzellen expremiert. So liegen die erfindungsgemäßen DNA-Sequenzen in einer bevorzugten Ausführungsform in Kombination mit Promotoren vor, die besonders aktiv in embryonalem Gewebe sind. Beispiele für solche Promotoren sind der USP-Promotor (Bäumlein et al. 1991, Mol. Gen. Genet. 225:459-467), der Hordein-Promotor (Brandt et al. 1985, Carlsberg Res. Commun. 50:333-345), sowie der Napin-Promotor, der ACP-Promotor und die FatB3- und FatB4-Promotoren, die dem auf dem Gebiet der pflanzlichen Molekularbiologie tätigen Fachmann wohl bekannt sind.

Gegebenenfalls können die Nukleinsäuresequenzen der Erfindung durch Enhancer-Sequenzen oder andere regulatorische Sequenzen ergänzt sein. Die regulatorischen Sequenzen beinhalten z.B. auch Signalsequenzen, die für den Transport des Genprodukts zu einem bestimmten Kompartiment sorgen.

Die vorliegende Erfindung betrifft auch Nukleinsäuremoleküle, die die erfindungsgemäßen Nukleinsäuresequenzen oder Teile davon enthalten, d.h. auch Vektoren, insbesondere Plasmide, Kosmide, Viren, Bakteriophagen und andere in der Gentechnik gängige Vektoren, die gegebenenfalls für den Transfer der erfindungsgemäßen Nukleinsäuremoleküle auf Pflanzen bzw. Pflanzenzellen eingesetzt werden können.

Bei den Pflanzen, die mit den erfindungsgemäßen Nukleinsäuremolekülen transformiert sind und in denen aufgrund der Einführung eines solchen Moleküls eines veränderte Menge an Fettsäuren synthetisiert wird, kann es sich im Prinzip um jede beliebige Pflanze handeln. Vorzugsweise ist es eine monokotyle oder dikotyle Nutzpflanze und besonders bevorzugt eine Ölpflanze. Als Beispiele sind hier insbesondere Raps, Sonnenblume, Sojabohne, Erdnuß, Kokos, Rüpsen, Baumwolle und Ölpalmen zu nennen. Weitere Pflanzen, die der Fettsäure- und Fettgewinnung dienen können oder als Nahrungsmittel mit erhöhtem Fettsäuregehalt nützlich sind, sind Lein, Mohn, Olive, Kakao, Mais, Mandel, Sesam, Senf und Ricinus.

Gegenstand der Erfindung sind ferner Vermehrungsmaterial von erfindungsgemäßen Pflanzen, beispielsweise Samen, Früchte, Stecklinge, Knollen, Wurzelstöcke etc., sowie Teile dieser Pflanzen wie Protoplasten, Pflanzenzellen und Kalli.

Bei den KAS 11-Sequenzen handelt es sich bevorzugt um Sequenzen, die aus *Brassica napus* isoliert worden sind.

Zur Erzeugung der erfindungsgemäßen Pflanzen bieten sich verschiedene Methoden an. Zum einen können Pflanzen bzw. Pflanzenzellen mit Hilfe herkömmlicher gentechnologischer Transformationsmethoden derart verändert werden, daß die neuen Nukleinsäuremoleküle in das pflanzliche Genom integriert werden, d.h. daß stabile Transformanten erzeugt werden. Zum anderen kann ein erfindungsgemäßes Nukleinsäuremolekül, dessen Anwesenheit und gegebenenfalls Expression in der Pflanzenzelle einen veränderten Fettsäuregehalt bewirkt, in der Pflanzenzelle bzw. der Pflanze als selbstreplizierendes System enthalten sein.

Zur Vorbereitung der Einführung fremder Gene in höhere Pflanzen stehen eine große Anzahl von Klonierungsvektoren zur Verfügung, deren Replikationssignale für *E.coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann in einer passenden Restriktionsschnittstelle in den Vektor eingeführt werden. Das erhaltene Plasmid wird dann für die Transformation von *E.coli*-Zellen verwendet. Transformierte *E. coli-Zellen* werden in einem geeigneten Medium gezüchtet und anschließend geerntet und lysiert, und das Plasmid wird wiedergewonnen. Als Analysenmethode zur Charakterisierung der gewonnenen Plasmid-DNA werden im allgemeinen Restriktionsanalysen, Gelelektrophoresen und weitere biochemischmolekularbiologische Methoden eingesetzt. Nach jeder Manipulation kann die Plasmid-DNA gespalten und gewonnene DNA-Fragmente mit anderen DNA-Sequenzen verknüpft werden.

Für die Einführung von DNA in eine pflanzliche Wirtszelle stehen eine Vielzahl bekannter Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, den direkten Gentransfer isolierter DNA in Protoplasten, die Elektroporation von DNA, die Einbringung von DNA mittels der biolistischen Methode sowie weitere Möglichkeiten.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide, wie z.B. pUC-Derivate, verwendet werden. Sollen aber aus derartig transformierten Zellen ganze Pflanzen regeneriert werden, ist die Anwesenheit eines selektierbaren Markergens notwendig. Dem Fachmann sind die Genselektionsmarker bekannt, und es stellt für ihn kein Problem dar, einen geeigneten Marker auszuwählen.

Je nach Einrührungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der im Ti- bzw. Ri-Plasmid enthaltenen T-DNA als Flankenbereich mit den einzuführenden Genen verbunden werden.

Werden für die Transformation Agrobakterien verwendet, muß die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E. coli* als auch in Agrobakterien replizieren. Sie enthalten ein Selektionsmarker-Gen und ein Linker oder Polylinker, welche von der rechten und linken T-DNA-Grenzregion eingerahmt werden. Sie können direkt in die Agrobakterien transformiert werden. Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet.

Die Verwendung von T-DNA für die Transformation von Pflanzenzellen ist intensiv untersucht und ausreichend in allseits bekannten Übersichtsartikeln und Handbüchern zur Pflanzentransformation beschrieben worden.

Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflarezenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biocide zur Selektion transformierter Zellen enthalten kann, wieder ganze Pflanzen regeneriert werden. Die Regeneration der Pflanzen erfolgt nach üblichen Regnerationsmethoden unter Verwendung bekannter Nährmedien. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplasten-Transformation sind ebenfalls bekannt und vielfach beschrieben.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biocid oder einem Antibiotikum wie Kanamycin, G418, Bleomycin, Hygromycin, Methotrexat, Glyphosat, Streptomycin, Sulfonyl-Harnstoff, Gentamycin oder Phosphinotricin und u.a. vermittelt. Der individuell gewählte Marker sollte daher die Selektion transformierter Zellen gegenüber Zellen, denen die eingeführte DNA fehlt, gestatten.

Die transfomierten Zellen wachsen innerhalb der Pflanze in der üblichen Weise. Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotyischen Eigenschaften. Von den Pflanzenzellen können Samen gewonnen werden.

Es sollten zwei oder mehrere Generationen herangezogen werden, um sicherzustellen, daß das phänotypische Merkmal stabil beibehalten und vererbt wird. Auch sollten Samen geerntet werden, um sicherzustellen, daß der entsprechende Phänotyp oder andere Eigenarten erhalten geblieben sind.

Ebenso können nach üblichen Methoden transgene Linien bestimmt werden, die für die neuen Nukleinsäuremoleküle homozygot sind, und ihr phänotypisches Verhalten hinsichtlich eines veränderten Fettsäuregehalts untersucht und mit dem von hemizygoten Linien verglichen werden.

Der Nachweis der Expression der erfindungsgemäßen Proteine mit KAS II Aktivität kann mit Hilfe herkömmlicher molekularbiologischer und biochemischer Methoden erfolgen. Dem Fachmann sind diese Techniken bekannt und er ist problemlos in der Lage, eine geeignete Nachweismethode zu wählen, beispielsweise eine Northern Blot-Analyse zum Nachweis KAS-spezifischer RNA bzw. zur Bestimmung der Höhe der Akkumulation von KAS-spezifischer RNA, eine Southern Blot-Analyse zur Identifizierung von für KAS II-kodierenden DNA-Sequenzen oder eine Western Blot-Analyse zum Nachweis des durch die erfindungsgemäßen DNA-Sequenzen kodierten Proteins, d.h. KAS II. Der Nachweis der enzymatischen Aktivität der KAS II kann anhand des Fettsäuremusters oder eines Enzymassays, wie z.B. in den nachfolgenden Beispielen beschrieben, bestimmt werden.

Während in den meisten Fällen eine Anreicherung mit bestimmten Fettsäuren in der Pflanzen, insbesondere im Samen oder der Frucht erstrebenswert ist, kann es auch wünschenswert sein, den Gehalt an bestimmten Fettsäuren zu reduzieren, bspw. aus diätetischen Gründen. In diesem Fall können die erfindungsgemäßen Sequenzen und Verfahren eingesetzt werden, um die Synthese von mittel- bzw. kurzkettigen Fettsäuren in Pflanzen zu unterdrücken. Die in diesem Fall einsetzbaren Methoden, insbesondere die der Antisense-Technik und die der Co-Suppressions-Strategie, sind dem auf dem Gebiet der pflanzlichen Biotechnologie tätigen Fachmann bekannt.

Die Erfindung basiert auf der erfolgreichen Isolierung neuer KAS II-Klone und der hier erstmals gelungenen Zuordnung konkreter Substratspezifitäten, die in den nachfolgenden Beispielen beschrieben werden.

Die nachfolgenden Beispiele dienen der Erläuterung der Erfindung.

### Beispiele:

### Beispiel 1: Klonierung eines cDNA-Klons für KAS II aus Brassica napus

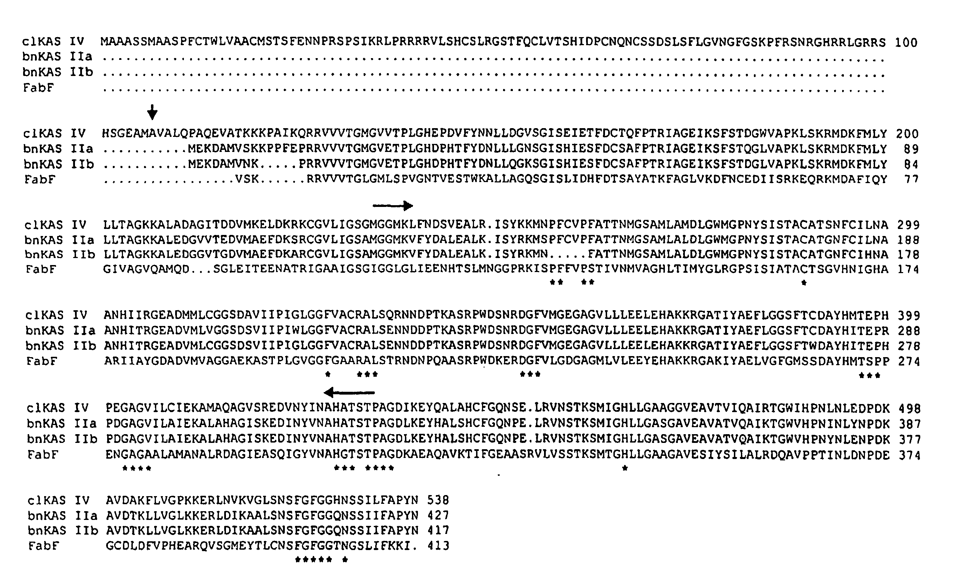

Gesamt-RNA würde aus Embryos von entwickelnden Samen von *Brassica napus* nach Voeltz et al. (1994) Plant Physiol. 106:785-786 isoliert und mRNA unter Verwendung von oligo-dT-Cellulose (Qiagen, Hilden, Deutschland) extrahiert. cDNA-Pools wurden von mRNA-Präparationen durch reverse Transkription mit einem oligo-dT-Adapter Primer (S'-AACTGGAAGAATTCGCGGCCGCAGGAAT₁₈-3') hergestellt. Basierend auf konservierten Regionen von KAS II kodierenden Genen aus *H. vulgare* (Wissenbach et al. (1994) Plant Physiol. 106:1711-1712), *R. communis,* (Knauf and Thompson (1996) U.S.-Patent 5,510,255) und *B. rapa* (Knauf and Thompson (1996) U.S.-Patent 5.510, 255) wurden degenerierte Oligonukleotide konstruiert, um PCR-Produkte von beiden cDNA-Templates zu erzeugen.Oligonukleotide "5kas2" (5'-ATGGGNGGCATGAAGGTNTT-3') und "3kas2" (5'-GTNGANGTNGCATGNGCATT-3') wurden entsprechend den Aminosäuresequenzen MGGMKVF und NAHATST (horizontale Peile in Abbildung 1) konstruiert. Die mittels dieser Oligonukleotidprimer erzeugten PCR-Produkte wurden sequenziert und anschließend die nachfolgenden Strategien verfolgt.

Für die Klonierung einer KAS II-cDNA aus *Brassica napus* (bnKASII), die das reife Protein kodiert, wurden semi-spezifische Oligonukleotide mit einer 5'-NdeI-Restriktionsschnittstelle konstruiert, basierend auf den bekannten Sequenzen von B. *rapa* KAS II (5'-Primer: 5'-CATATGGARAARGAYGCNATGGT-3', 3'-Primer: 5'-TCANTTGTANGGNGCRAAAA-3'), und die entstandene bnKASIIa-cDNA wurde in die NdeI-Restriktionschnittstelle des pET15b-Expressionsvektors (Novagen, Madison Wi, USA) kloniert.

Zwei verschiedene Klone wurden erhalten, bnKASIIa und bnKASIIb, deren abgeleiteten Aminosäuresequenzen 97,4 % Identität zeigten (siehe Abbildung 1). Die DNA-Sequenz des cDNA-Klons bnKASIIa ist in SEQ ID:No. 3 gezeigt, die DNA-Sequenz des cDNA-Klons bnKASIIb in SEQ ID:No. 5. Die abgeleiteten Aminosäuresequenzen sind in SEQ ID No. 4 bzw. SEQ ID:No. 6 gezeigt. Der Klon bnKASIIb zeigt Lücken bei den Positionen 10-14 und 146-150, wobei die erste Lücke ebenfalls in der *B. rapa*-Sequenz vorhanden ist, und die zweite Lücke für den Verlust des Peptids PFCNP, ein Motiv, das in sämtlichen anderen soweit bekannten KASII-Scquenzen anwesend ist, verantwortlich. Für *E.coli* KASII ist dieses Motiv für die Bildung der potentiellen Substratbindungstasche essentiell (* in Abbildung 1), welche das Cystein der aktiven Site umgibt (Huang et al. (1998) EMBO J. 17:1183-1191).

Klon bnKASIIa kodiert für ein Polypeptid von 427 Aminosäuren, welche eine Identität von 65 % mit den Enzymen des KASI-Typs von *Rhizinus communis* (L 13242), *Arabidopsis thaliana* (U24177) und *Hordeum vulgare* (M760410) und eine Identität von über 85 % mit Enzymen des vermutlichen KASII-Typs von *R. communis* (Knauf and Thompson, supra) und *H. vulgare* (Z34268 und Z34269) aufweist.

### Beispiel 2: Klonierung einer cDNA für KASIV aus Cuphea lanceolata

Die Erzeugung von PCR-Produkten erfolgte wie in Beispiel 1 beschrieben.

Für die Klonierung von full lenght-cDNA von *C. lanceolata* wurden neue spezifische Oligonukleotide gemäß der Sequenzinformation des ersten PCR-Fragments, erhalten wie oben beschrieben, konstruiert, um damit 3'- und 5'-RACE (Rapid amplification of cDNA ends) durchzuführen. Für die Herstellung von rekombinantem Protein wurde die das reife Protein kodierende c1KASIV-cDNA durch Einführung einer NdeI-Restriktionsschnittstelle am Methionin¹⁰⁶ unter Einsatz der PCR-Technik konstruiert (siehe Abbildung 1). Die modifizierte cDNA wurde in die NdeI-Schnittstelle des Histag-Expressionsvektors pET15b eingefügt. Sämtliche PCR-Reaktionen wurden unter Verwendung der Pfu DNA-Polymerase (Stratagene, Heidelberg, Deutschland) durchgeführt.

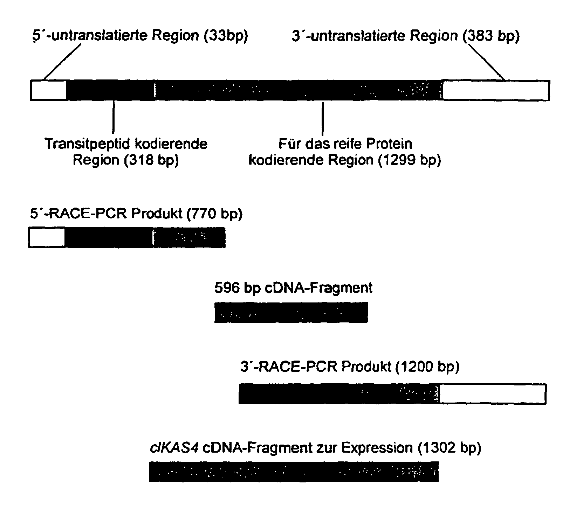

Sequenzvergleiche sämtlicher erhaltener Klone zeigten, daß die ersten 435 Basenpaare und die letzten 816 Basenpaare des cDNA-Fragments (clKASIVm), die das reife Protein kodieren, mit den entsprechenden Teilen eines 5'-RACE-Fragments bzw. einem 3'-RACE-Fragment identisch waren, weshalb eine mit clKASIV (SEQ ID:No. 1) bezeichnete theoretische full lenght-cDNA abgeleitet wurde (Figur 2). Diese clKASIV-cDNA umfaßt eine 33 Basenpaar langes 5'-untranslatierte Region, eine 1617 Basenpaar lange kodierende Region und eine 383 Basenpaare umfassende 3'-untranslatierte Region. Die abgeleitete Aminosäuresequenz der clKASIV für das reife Protein zeigte eine Identität von über 94 % mit den kürzlich veröffentlichten KASIV-Sequenzen von *C, wrightii* (Slabaugh et al. (1998) Plant J. 13:611-620, *C, hookeriana* und *C pulcherrima* (Dehesh et al. (1998) Plant J. 15:383-390). Die Identitäten mit Sequenzen vom KASII-Typ sowie mit bnKASIIa liegen bei ungefähr 85 %, während die Identitäten mit Sequenzen vom KASI-Typ ungefähr 65 % betragen.

### Beispiel 3: Expression und Reinigung rekombinanter KASII- und KASIV-Enzyme

Frisch transformierte *E. coli* BL21 (DE3)-Zellen wurden bei 25 °C in 21 TB-Medium mit 50 g/ml Ampicillin kultiviert. Bei einer Zelldichte von 0,7 bis 0,8 OD₆₀₀ wurde die Expression der rekombinanten Proteine durch Zugabe von Isopropylthiogalactosid bis zu einer Endkonzentration von 20 µM induziert und das Zellwachstum für eine weitere Stunde fortgesetzt. Die Zellen wurden durch Zentrifugation geerntet und über Nacht bei -20° C gelagert.

Die Zellen wurden für 30 Minuten auf Eis in 20 ml folgender Lösung lysiert: 50 mM Natriumphosphat, pH 7,6, 10% (v/v) Glycerol, 500 mM Natriumchlorid, 10 mM Imidazol, 0,1 mM Phenylmethylsulfonylfluorid, 100 µg/ml Lysozym und 2,5 U/ml Benzonase. Die restlichen Zellen wurden durch Sonifikation (3 x 10 s) aufgebrochen und die gesamte lösliche Fraktion auf eine Ni-NTA-Superflow-Säule (5 ml Qiagen, Hilden, Deutschland) geladen. Unspezifisch gebundene Proteine wurden durch Waschen mit 40 ml von 50 mM Natriumphosphat, pH 7,6, enthaltend 500 mM Natriumchlorid, 10 % (v/v) Glycerol und 50 mM Imidazol, entfernt. In einem zweiten Waschschritt wurde die Säule mit 20 ml von 50 mM Natriumphosphat, pH 7,6, enthaltend 10 % (v/v) Glycerol und 50 mM Imidazol, behandelt, um das Natriumchlorid zu entfernen. Schließlich wurden die rekombinanten Enzyme mit dem gleichen Puffer eluiert, der allerdings für diesen Schritt 250 mM Imidazol enthielt. Die Fraktionen wurden bis zur Verwendung bei -70 °C gelagert.

Die Ausbeute betrug ca. 250 µg lösliches rekombinantes Enzym pro Liter Kultur.

SDS-PAGE zeigte, daß die affinitätsgereinigten Enzyme KASII und KASIV im wesentlichen frei von Proteinkontaminationen waren. Die rekombinanten Enzyme : einschließlich des N-terminalen fusionierten His-tags, haben vorausgesagte Molekulargewichte von 48,0 kDa (bnKASIIa) und 48,5 kDa (cIKASIV), was in guter Übereinstimmung mit dem Molekulargewicht von 47 kDa in der SDS-PAGE ist. Die Authentizität beider Proteine wurde durch Antikörperfärbung mit anti-His-tag-Antikörpern verifiziert.

### Beispiel 4: Herstellung von Acyl-ACP-Substraten

ACP von *E. coli* wurde von Sigma (Deisenhofen, Deutschland) bezogen und durch Anionenaustausch-FPLC auf Mono Q, wie bei Kopka et al. (1993) Planta 191:102-111 beschrieben, gereinigt. C₆- bis C₁₆-.Acyl-ACPs wurden enzymatisch aus *E. coli* ACP unter Verwendung einer Acyl-ACP-Synthethase aus *Vibrio harveyi* (Shen et al. (1992) Anal.Biochem. 204:34-39) synthetisiert. Butyryl-ACP wurde nach Cronan and Klages (1981) Proc. Natl. Acad. Sci. USA 78:5440-5444) chemisch hergestellt und weiter nach Brück et al. (1996) Planta 198:271-278 gereinigt. Die Reinheit und Konzentration der Acyl-ACP-Stocklösungen wurden durch konformationell sensitive Gelelektrophorese in 20 % Acrylamid-Gelen, enthaltend 2,5 M Harnstoff, gefolgt durch Visualisierung mit Coomassie Blue und densitometrische Quantifizierung bestimmt, wobei gereinigtes ACP bekannter Konzentrationen als Standard diente. Malonyl-ACP wurde von ACP und Malonyl-CoA unter Verwendung einer partiell gereinigten Malonyl-CoA:ACP-Transacylase (MAT) von *C. lanceolata-Samen* enzymatisch synthetisiert (Brück et al. (1994) J.Plant Physiol. 143:550-555). Das Reaktionsgemisch (0.5 ml) enthielt 100 mM Natriumphosphat, pH 7,6, 40 µM gereinigtes ACP, 80 µM [2-¹⁴C]Malonyl-CoA (0,74 MBq/mmol), 150 µl MAT-Präparation (entsprechend 0,22 nkat) und 2 mM Dithiothreitol (DTT). Für die vollständige Reduktion wurde ACP mit DTT für 15 Minuten bei 37 °C vor Zugabe der anderen Bestandteile vorinkubiert. Die Reaktion wurde für 10 Minuten bei 37 °C zugelassen und durch die Zugabe von 55 µl von 100 % (w/v) Trichloressigsäure (TCA) gestoppt. Nach Inkubation auf Eis für mindestens 10 Minuten wurde das Gemisch zentrifugiert (16.000 x g, 5 Minuten, 4 °C), und der das nichtreagierte Malonyl-CoA enthaltende Überstand wurde abgenommen und verworfen. Das Präzipitat wurde mit 200 µl von 1 % (w/v) TCA gewaschen, wie oben zentrifugiert und in 50 mM 2-[N-Morpholino]Ethansulfonsäure, pH 6,8, gelöst und in Aliquots bei -20 °C gelagert. Die Konzentration der [2-¹⁴C]Malonyl-ACP-Präparation wurde anhand von Flüssigscintillationsspektometrie-Daten bestimmt.

### Beispiel 5: Enzymassay

Die Substratspezifitäten der rekombinanten KASII- und KASIV-Enzyme wurden durch die Inkorporation von Radioaktivität von [2-¹⁴C]Malonyl-ACP in die Kondensationsprodukte untersucht. Der Ansatz (50 µl) enthielt 100 mM Natriumphosphat, pH 7,6, 10 µM Acyl-ACP von spezifischer Kettenlänge, 7,5 µM [2-¹⁴C]Malonyl-ACP (0,74 MBq/mmol), 2 mM NADPH, 2 mM DTT, 0,6 µkat von affinitätsgereinigtem rekombinanten GST-β-Ketoacyl-ACP-Reduktase-Fusionsprotein von *C. lanceolata* (Klein et al. (1992) Mol. Gen. Genet. 233:122-128) und 2 µg der rekombinanten KASII/IV-Präparation. Die β-Hydroxyacyl-ACPs, die synthetisiert wurden, wurden präzipitiert, gewaschen und wie bei Winter et al. (1997) Biochem. J. 321:313-318 beschrieben gelöst und anschließend durch eine 2,5 M Harnstoff-PAGE aufgetrennt. Nach dem Transfer auf eine Immobilon P-Membran durch Elektroblotting bei 0,8 mA/cm² für 1 h wurden die Reaktionsprodukte durch Autoradiographie nach fünftägiger Exposition auf einem Röntgenfilm (Hyperfilm MP, Amersham, Braunschweig, Deutschland) sichtbar gemacht.

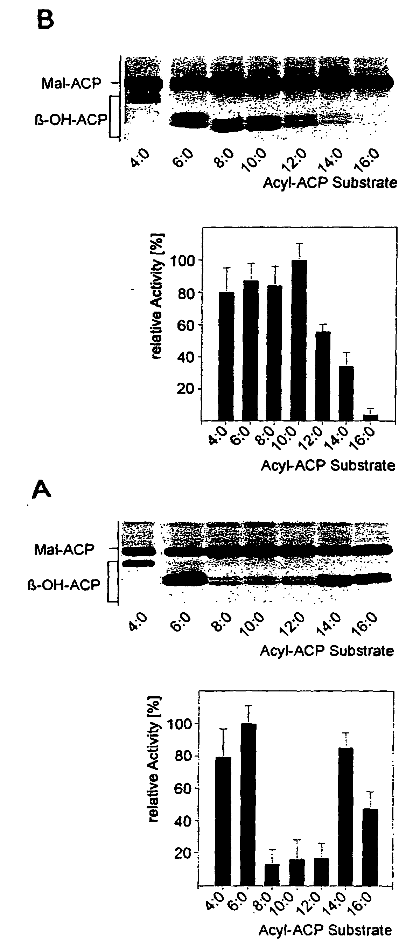

In den Essays wurde gesättigtes Acyl-ACP (C₄ bis C₁₆) zusammen mit [2-¹⁴C]Malonyl-ACP zu dem Reaktionsgemisch hinzugegeben und für 10 Minuten inkubiert. Die Inkorporation der Radioaktivität aus [2-¹⁴C]Malonyl-ACP in das β-Ketoacyl-ACP-Produkt, das für die Analyse zu β-Hydroxyacyl-ACP reduziert wurde, wurde bestimmt. Die Ergebnisse zeigen verschiedene Merkmale für zwei phylogenetisch eng verwandte Kondensierungsenzyme. Während für die bnKASIIa-Katalyse die Elongation von C₁₄und C₁₆-ACPs - wie für langkettige Fettsäuren produzierende Pflanzen zu erwarten - beobachtet werden konnte, wurde darüberhinaus auch die Elongation von kurzkettigen Acyl-ACPs bis C₆ festgestellt (siehe Abbildung 3A).

Die Untersuchung der clKASIV-Katalyse zeigte eine Kurzketten-spezinsche Kondensierungsaktivität sowie, im Unterschied zu KASIIa, eine nachfolgende Mittelketten-spezifische Kondensierungsaktivität bis zu C₁₀ (siehe Abbildung 3B). Darüberhinaus war die Sensitivität von clKASIV gegenüber Cerulenin höher (IC₅₀ = 20 µM) im Vergleich zu bnKASIIa, aber dennoch deutlich niedriger als die für Enzyme vom KASI-Typ bekannte Sensitivität, welche in Anwesenheit von 5 µM Cerulenin bereits vollständig Inaktiviert sind (Shimakata and Stumpf (1982) Proc. Natl. Acad. Sci. USA 79:5808-5812). Da von Cerulenin angenommen wird, daß es ein Substratanalogon für C₁₂-ACP ist (Morisaki et al. (1993) Eur. J. Biochem. 211:111-115), ist es nachvollziehbar, daß die Spezifität der KASIV für mittelkettige Acyl-ACPs dieses Enzym sensitiver gegenüber Cerulenin macht als KASII.

Zusammenfassend konnte hier somit erstmals gezeigt werden, daß zwar sowohl KASII als auch KASIV in der Lage sind, kurzkettige Acyl-ACP-Produkte (C₄ und C₆) zu elongieren, daß aber nur KASIV die Elongation von Acyl-ACP von C₈ - C₁₂ katalysiert. Andererseits zeigt nur KASII eine hohe Kondensierungsaktivität für die Substrate C₁₄-ACP und C₁₆-ACP, während der KASIV diese Aktivitäten fehlen.

### Beschreibung der Figuren:

### Abbildung 1:

Alignment der Aminosäuresequenzen von bnKASIIa, bnKASIIb und cIKASIV. abgeleitet von den jeweiligen Nukleotidsequenzen. Die für das Design der degenerierten Primer 5kas2 und 3kas2 verwendeten Aminosäuren sind durch horizontale Pfeile markiert. Ein vertikaler Pfeil markiert den vermutlichen Start der reifen clKAS. Die *E. coli* KASII (FabF) wurde von der Genbank-Accession Nummer P39435 abgeleitet.

### Abbildung 2:

Schema für die Klonierung von clKAS4.

### Abbildung 3:

Substratspezifität der gereinigten rekombinanten bnKASIIa (A) und clKASIV (B). Die Reaktionsprodukte wurden durch 2,5 M Harnstoff-PAGE aufgetrennt, auf eine PVDF-Membran geblottet und durch Autoradiographie sichtbar gemacht (jeweils oberer Teil der Abbildungen A und B). Die zwei Banden von Reaktionsprodukten repräsentieren *E. coli* ACP-Isoformen, wie sie bereits früher beobachtet wurden (Winter et al. (1997) supra). Die Werte zeigen Durchschnittswerte ± Standardabweichung (n = 4, für das Substrat C₄ n = 2). Mal-ACP = MalonylACP; β-OH-ACP = β-Hydroxyacyl-ACP.

DNA- und Aminosäuresequenzen für β-Ketoacyl-ACP-Synthase (in 5' -> 3'-Richtung bzw. vom N-Terminus zum C-Terminus
1) SEQ ID:No. 1 - β-Ketoacyl-ACP-Synthase IV aus *Cuphea lanceolata* DNA-Sequenz des cDNA-Klons clKAS4
2) SEQ ID:No. 2 - β-Ketoacyl-ACP-Synthase IV aus *Cuphea lanceolata* Aminosäuresequenz des cDNA-Klons clKAS4
3) SEQ ID:No. 3 - β-Ketoacyl-ACP-Synthase II aus *Brassica napus* DNA-Sequenz des cDNA-Klons bnKAS2a
4) SEQ ID:No. 4 - β-Ketoacyl-ACP-Synthase II aus *Brassica napus* Aminosäuresequenz des cDNA-Klons bnKAS2a
5) SEQ ID:No. 5 - β-Ketoacyl-ACP-Synthase II aus *Brassica napus* DNA-Sequenz des cDNA-Klons bnKAS2b
6) SEQ ID:No. 6 - β-Ketoacyl-ACP-Synthase II aus *Brassica napus* Aminosäuresequenz des cDNA-Klons bnKAS2b
7) SEQ ID:No. 7 - β-Ketoacyl-ACP-Synthase I aus *Cuphea lanceolata* DNA-Sequenz des cDNA-Klons CIKAS1
8) SEQ ID:No. 8 - β-Ketoacyl-ACP-Synthase I aus *Cuphea lanceolata* Aminosäuresequenz des cDNA-Klons CIKAS 1

## Patentansprüche

1. Verfahren zur Erhöhung des Gehalts an kurzkettigen Fettsäuren mit bis zu 6 C-Atomen in Pflanzensamen, umfassend die Schritte:
a) Herstellung einer Nukleinsäuresequenz, mindestens umfassend die folgenden Bestandteile, die in der 5'-3'-Orientierung aneinandergereiht sind:
- ein in Pflanzen, insbesondere in embryonalem Gewebe, aktiver Promotor,
- mindestens eine Nukleinsäuresequenz, die für ein Protein mit der Aktivität einer β-Ketoacyl-ACP-Synthase II mit Substratspezifität für kurzkettige Acyl-ACPs oder ein funktionell aktives Fragment davon kodiert und
- gegebenenfalls ein Terminationssignal für die Termination der Transkription und die Addition eines poly-A-Schwanzes an das entsprechende Transkript, sowie ggf. davon abgeleitete DNA-Sequenzen;
b) Übertragung der Nukleinsäuresequenz aus a) auf pflanzliche Zellen und
c) ggf. Regeneration vollständig transformierter Pflanzen und, falls erwünscht, Vermehrung der Pflanzen.

2. Verfahren nach Anspruch 1, worin die für ein Protein mit der Aktivität einer β-Ketoacyl-ACP-Synthase II oder ein funktionell aktives Fragment davon kodierende Nukleinsäuresequenz aus *Brassica napus* stammt und/oder eine Sequenz umfassend SEQ:ID No. 3 oder SEQ:ID No. 5 oder ein Fragment davon ist welches fur ein funktionell aktives Fragment einer β-Ketoacyl-ACP-Synthase II kodiert.

3. Verfahren nach Anspruch 1 oder 2, worin zusätzlich die endogene Aktivität der β-Ketoacyl-ACP-Synthase I unterdrückt wird, z.B. durch antisense-Expression oder Co-Suppression.

4. Verfahren nach einem der Ansprüche 1 bis 3, worin zusätzlich eine für Thioesterase, insbesondere eine mittelkettenspezifische oder kurzkettenspezifische Thioesterase kodierende Nukleinsäuresequenz übertragen wird.

5. Verwendung des Verfahrens gemäß einem der Ansprüche 1 bis zur Herstellung einer Pflanze zur Gewinnung von Pflanzenöl mit erhöhtem Fettsäuregehalt.

## Claims

1. A method for increasing the content of short-chain fatty acids comprising up to 6 carbon atoms in plant seeds, comprising the steps:
a) producing a nucleic acid sequence comprising at least the following components, which are successively arranged in 5'-3' orientation:
- a promoter which is active in plants, especially in embryonal tissue,
- at least one nucleic acid sequence which codes for a protein having the activity of a β-ketoacyl-ACP-synthase II, which has substrate specificity for short-chain acyl-ACPs, or which codes for a functionally active fragment thereof, and
- optionally, a termination signal for transcription termination and addition of a poly(A) tail to the corresponding transcript, as well as, optionally, DNA sequences derived therefrom;
b) transferring the nucleic acid sequence from a) to plant cells, and
c) optionally, regenerating fully transformed plants and, if desired, propagating the plants.

2. The method according to claim 1, wherein the nucleic acid sequence coding for a protein, which has the activity of a β-ketoacyl-ACP-synthase II, or coding for a functionally active fragment thereof, is originated from *Brassica napus* and/or represents a sequence comprising SEQ ID NO. 3 or SEQ ID NO: 5 or a fragment thereof, which codes for a functionally active fragment of a β-ketoacyl-ACP-synthase II.

3. The method according to claim 1 or 2, wherein additionally the endogenous activity of the β-ketoacyl-ACP-synthase I is suppressed e.g., by antisense expression or co-suppression.

4. The method according to any one of claims 1 to 3, wherein additionally a nucleic acid sequence coding for a thioesterase, especially a medium-chain-specific or short-chain-specific thioesterase, is transferred.

5. Use of the method according to any one of claims 1 to 4 for the production of a plant in order to obtain vegetable oil having an increased fatty acid content.

## Revendications

1. Procédé pour l'augmentation de la teneur en acides gras à courte chaîne présentant jusqu'à 6 atomes de carbone dans des semis de plante, comprenant les étapes de :
a) fabrication d'une séquence d'acides nucléiques comprenant au moins les composants suivants qui s'ajoutent l'un à côté de l'autre selon une orientation 5'-3' :
- un promoteur actif dans des plantes, notamment dans un tissu embryonnaire ;
- au moins une séquence d'acides nucléiques qui code pour une protéine avec l'activité d'une β-cétoacyl-ACP-synthase II présentant une spécificité de substrat pour les acyl-ACP à courte chaîne ou code pour un fragment actif d'un point de vue fonctionnel et
- le cas échéant, un signal de terminaison pour la terminaison de la transcription et l'ajout d'une séquence poly(A) au niveau du transcript correspondant ainsi, le cas échéant, que des séquences d'ADN dérivées de celui-ci ;
b) la transmission de la séquence d'acides nucléiques à partir de a) à des cellules végétales et
c) la régénération, le cas échéant, de la plante entièrement transformée et si nécessaire la multiplication des plantes.

2. Procédé selon la revendication 1, dans lequel la séquence d'acides nucléiques codant pour une protéine présentant l'activité d'une β-cétoacyl-ACP-synthase II ou un fragment actif fonctionnel de celle-ci qui provient de *Brassica napus* et/ou une séquence comprenant SEQ : ID No. 3 ou SEQ : ID No. 5 ou qui est un fragment lequel code pour un fragment actif d'un point de vue fonctionnel d'une β-cétoacyl-ACP-synthase II.

3. Procédé selon la revendication 1 ou 2, dans lequel l'activité endogène de la β-cétoacyl-ACP-synthase I est en plus réprimée, par exemple par l'expression antisens ou la cosuppression.

4. Procédé selon l'une quelconque des revendications 1 à 3, dans lequel une séquence d'acides nucléiques codant pour une thioestérase notamment pour une thioestérase spécifique à chaîne intermédiaire ou spécifique à chaîne courte est en plus transmise.

5. Utilisation du procédé selon l'une quelconque des revendications 1 à 4, pour la fabrication d'une plante par obtention d'une huile végétale présentant une teneur élevée en acides gras.
